(19) Europäisches Patentamt
European Patent Office
Office européen des brevets

(11) Veröffentlichungsnummer : **0 121 671 B2**

# (12) NEUE EUROPÄISCHE PATENTSCHRIFT

(45) Veröffentlichungstag der neuen Patentschrift : **06.11.91 Patentblatt 91/45**

(51) Int. Cl.[5] : **C07C 37/60**, C07C 39/08

(21) Anmeldenummer : **84101170.3**

(22) Anmeldetag : **06.02.84**

## (54) Verfahren zur Herstellung von Dihydroxybenzolen.

Die Akte enthält technische Angaben, die nach dem Eingang der Anmeldung eingereicht wurden und die nicht in dieser Patentschrift enthalten sind.

(30) Priorität : **11.03.83 DE 3308726**

(43) Veröffentlichungstag der Anmeldung : **17.10.84 Patentblatt 84/42**

(45) Bekanntmachung des Hinweises auf die Patenterteilung : **20.05.87 Patentblatt 87/21**

(45) Bekanntmachung des Hinweises auf die Entscheidung über den Einspruch : **06.11.91 Patentblatt 91/45**

(84) Benannte Vertragsstaaten : **AT BE CH DE FR GB IT LI LU NL SE**

(56) Entgegenhaltungen :
DE-A- 2 348 957
DE-A- 2 410 758
FR-A- 2 201 279

(56) Entgegenhaltungen :
US-A- 2 616 791
GMELIN, Handbuch der Anorganischen Chemie, 8. Ausgabe, 9, (1960), Seiten 514/515
Collection Czechoslov. Chem. Comm., Band 21 (1956), Seiten 1073 bis 1075
J.A:C:S: Band 51 (1929), Seiten 1457/1458
Landolt-Börnstein, Physikalisch-chemische Tabellen (1923), Seite 2105
Chemiker-Zeitung 1941, 65, 194 - 195
"H2O2 in der Organischen Synthese", Broschüre der Firma Peroxid-Chemie GmbH (1981)
WEAST, Handbook of Chem. & Phys., 6th Ed., CRC-Press, 1988, Seiten D 69, D87, und D 88
Landold Börnstein Band II. 6. Auflage, 1961, Seite 527

(73) Patentinhaber : **Degussa Aktiengesellschaft**
**Weissfrauenstrasse 9**
**W-6000 Frankfurt am Main 1 (DE)**

(72) Erfinder : **Drauz, Karlheinz, Dr. Dipl.-Chem.**
**Flurstrasse 5**
**W-6463 Freigericht 1 (DE)**
Erfinder : **Kleemann, Axel, Dr. Dipl.-Chem.**
**Greifenhagenstrasse**
**W-6450 Hanau 9 (DE)**

EP 0 121 671 B2

**Beschreibung**

Die Erfindung betrifft die Herstellung von Dihydroxybenzolen sowie deren Monoäthern durch Kernhydroxylierung der entsprechenden Phenole oder Phenoläther mit Wasserstoffperoxid.

Wichtige Dihydroxybenzole sind Abkömmlinge von Phenol, Naphtholen, aber auch von Anthracen oder Phenanthren. Sie lassen sich z.B. bei der Herstellung von Farbstoffen, in der Kunststoffproduktion, als Photochemikalie, bei der Herstellung von Pflanzenschutzmitteln verwenden. So wird z.B. Hydrochinon als Para-Hydroxylierungsprodukt von Phenol als Photochemikalie; Brenzcatechin als das entsprechende Orthoprodukt für Pflanzenschutzmittel eingesetzt. Verschiedene Anwendungsgebiete, wie z.B. als Antioxydantien, sind den Dihydroxyphenolen gemeinsam.

Ihre Herstellung war daher schon lange Gegenstand eingehender Untersuchungen. Die Hydroxylierung wurde sowohl mit Wasserstoffperoxid selbst, wie auch mit Hydroperoxiden, Peroxiden oder auch Persäuren, wie z.B. Perameisen- oder Peressigsäure, durchgeführt. Doch war Wasserstoffperoxid, da es am leichtesten zugänglich war, bevorzugt, da mit Percarbonsäuren, Hydroperoxiden und Peroxiden Nebenreaktionen auftraten. (EP-A 0 027 5993).

Stets war bei diesen Hydroxylierungen ein Katalysator anwesend. Dieser Katalysator konnte ein Metalloid, wie Schwefel, Selen, Tellur, Phosphor, Arsen oder Antimon in elementarer Form sein (DE-OS 23 48 957), oder man verwendete Borverbindungen (DE-PS 1 543 830).

Verschiedene Verfahren arbeiteten mit Übergangselementen in Form ihrer Ionen (DE-OS 21 62 552), besonders mit Eisenionen (DE-OS 2162 589 oder DE-PS 24 07 398) oder Kobaltionen (DE-AS 23 41 743) oder auch mit den entsprechenden Oxiden (US-PS 2,395,638).

Ausserdem wurden starke Säuren, wie Schwefelsäure, Sulfonsäuren (DE-OS 21 38 735, DE-AS 24 10 742, DE-AS 24 10 758, DE-AS 24 62 957) oder Gemische aus Schwefel- und Phosphorsäure (DE-OS 21 38 735) eingesetzt, bzw. wurden in dieser letztgenannten Offenlegungsschrift organische Säuren, wie u.a. Trichloressigsäure oder Weinsäure, genannt.

Die schon genannten Percarbonsäuren dienten ebenfalls als Katalysatoren (FR-PS 1 479 354). Bei den Katalysatoren handelte es sich in allen genannten Fällen um feste oder flüssige Substanzen. Wasserstoffperoxid - als bevorzugtes Oxydationsmittel - wurde meist in wässriger Lösung verschiedener Konzentrationen bis hin zu sehr hohen, explosionsgefährlichen Konzentrationen eingesetzt; so arbeitete das Verfahren nach der DE-PS 20 64 497 mit Lösungen, die nur noch 5 Gew.-% Wasser enthielten, aber selbst bei diesem höchstkonzentriertem Wasserstoffperoxid lag die Ausbeute an Dihydroxyderivaten nur bei 70 % und sank entsprechend der Verdünnung des Wasserstoffperoxids erheblich ab.

Hinzu kam, dass in diesem wie auch in anderen Verfahren mit einem sehr grossen Überschuss an dem zu hydroxylierenden Phenol gearbeitet werden musste, um überhaupt die oben angegebene Ausbeute zu erhalten. Wurde dieser Überschuss gesenkt, z.B. von 20 Mol auf 10 Mol pro Mol Wasserstoffperoxid, so sank trotz hoher Konzentration an Wasserstoffperoxid die Ausbeute drastisch.

Bekanntlich erfordern aber derartige Überschüsse an einer Reaktionskomponente, die ja zurückgeführt werden muss, zusätzliche technische Aufwendungen; vor allem hinsichtlich der Grösse der einzusetzenden Apparaturen.

Da man immer bemüht ist, grosse Überschüsse an einer Komponente nach Möglichkeit zu vermeiden, wurde versucht, den Einsatz wässriger Lösungen von Wasserstoffperoxid zu umgehen. So wurden schon verschiedentlich Lösungen von Wasserstoffperoxid in organischen Lösungsmitteln verwendet. Man arbeitete z.B. nach dem Verfahren der DE-PS 24 10 758 bevorzugt mit Wasserstoffperoxidlösungen in Abkömmlingen der Phosphor- oder Phosphonsäure, und zwar in Gegenwart einer starken Säure, wie Schwefelsäure (100%ig) oder Fluorsulfonsäure.

Diese hochkonzentrierten starken Säuren haben aber den Nachteil, dass ihre Abtrennung aus dem Reaktionsgemisch Schwierigkeiten bereitete (DE-AS 26 58 943), vor allem, da ihre Konzentration im Reaktionsgemisch die Reaktionsdauer erheblich beeinflusst.

Die Überschüsse an Phenolen waren zwar gegenüber denen im Verfahren der DE-AS 20 64 497 etwas vermindert, aber dies wog den Nachteil durch die starken Säuren nicht auf.

Eine zusätzliche Schwierigkeit beim Verfahren der DE-PS 24 10 758 bei der Aufarbeitung des Reaktionsgemisches wurde durch die Anwesenheit des nach der Reaktion mit Wasserstoffperoxid gebildeten Wassers hervorgerufen. Da die eingesetzten Lösungsmittel für Wasserstoffperoxid zum Teil höher siedeten als die eingesetzten Phenole und diese oft - vor allem auch Phenol selbst - mit Wasser Azeotrope bildeten. deren Siedepunkte unter denen der organischen Lösungsmittel lagen, war eine einwandfreie Abtrennung der überschüssigen Phenole aus dem Reaktionsgemisch äusserst problematisch.

Man ging daher andere Wege und versuchte, zunächst einmal ohne Katalysator, d.h vor allem ohne die starken Säuren, auszukommen. Da die Katalysatoren in erster Linie für die Aktivierung von Wasserstoffperoxid

notwendig waren, wurde im Verfahren der DE-AS 26 58 943 mit organischen Lösungen von Percarbonsäuren gearbeitet. Ein zusätzlicher Katalysator wurde nicht verwendet.

Ganz abgesehen davon, dass das genannte Verfahren eine vollständige Anlage zur Herstellung einer organischen Percarbonsäure, die zunächst aus Wasserstoffperoxid und Carbonsäure gewonnen und darauf durch Extraktion dieser sog. "Gleichgewichtssäure" aus ihrem wäßrigen Medium hergestellt wird. voraussetzt. hatte sich gezeigt, dass eine angeblich gute Selektivität und gute Ausbeute nur durch Anwesenheit zusätzlicher Persäurestabilisatoren möglich war (DE-OS 23 64181; EP-A 0 027 593).

Gemäß dem Verfahren der DE-OS 23 48 957 werden Metalloide, darunter Schwefel, in einer Menge von 0,001 bis 1 Grammatom je Mol Wasserstoffperoixd als Katalysator eingesetzt. Um eine befriedigende Ausbeute zu erzielen, bedarf es beispielsgemäß einer Menge von 0,187 Mol Schwefel pro Mol Wasserstoffperoxid und zusätzlich der Verwendung eines komplexbildenden Mittels.

Nach den Verfahren zur Kernhydroxylierung von Phenolen mit Wasserstoffperoxid und Mineralsäuren als Katalysator (vgl. DE-OS 20 64 497, DE-OS 24 10 742, DE-OS 24 10 758 und DE-OS 24 62 957) wird der Katalysator, wie auch aus den Beispielen hervorgeht, vorzugsweise in einer Menge von 0,1 bis 0,5 Mol pro Mol Wasserstoffperoxid eingesetzt.

Ziel der Erfindung war es daher, die Kernhydroxylierung von Phenol und substituierten Phenolen oder deren Äthern mit Wasserstoffperoxid in Gegenwart von Katalysatoren in technisch einfacher Weise und mit sehr guten Ausbeuten durchzuführen.

Gefunden wurde nun ein Verfahren zur Herstellung von Kernhydroxylierungsprodukten der entsprechenden Phenole oder Phenoläther durch Umsetzung derselben mit organischen Lösungen von Wasserstoffperoxid, die einen Wassergehalt von höchstens 1 Gew.-%, bevorzugt weniger als 0,5 Gew.-%, haben und mit Lösungsmitteln hergestellt worden sind, die mit Wasser keine oder nur solche Azeotrope bilden, die oberhalb des Siedepunktes von Wasserstoffperoxid sieden, bezogen auf Normaldruck, in Gegenwart eines Katalysators bei einem Molverhältnis von Phenol bzw. Phenoläther zu Wasserstoffperoxid im Bereich von 5 bis 20, das dadurch gekennzeichnet ist, das man die Umsetzung mit Schwefeldioxid als Katalysator vornimmt, wobei man 0,0005 bis 0,01 Mol Schwefeldioxid auf 1 Mol Wasserstoffperoxid verwendet. Die Unteransprüche richten sich auf bevorzugte Ausführungsformen des Verfahrens.

Obgleich bekannt ist, daß Schwefeldioxid mit Wasserstoffperoxid Schwefelsäure bildet, entfaltet Schwefeldioxid im anspruchsgemäßen Reaktionssystem überraschenderweise eine wesentlich höhere katalytische Wirksamkeit als die äquivalente Menge Schwefelsäure.

Schwefeldioxid wird sowohl in gasförmigem Zustand wie in einem beliebigen Lösungsmittel gelöst eingesetzt. Dieses Lösungsmittel soll mit Wasserstoffperoxid wie mit Schwefeldioxid keine störenden Reaktionen eingehen. So kommen beispielsweise in Frage:

Dialkyläther, Ester der Phosphor- oder Phosphonsäure oder Alkyl- oder Cycloalkylester von gesättigten aliphatischen Carbonsäuren, die eine Gesamtkohlenstoffzahl von 4 - 8 besitzen. Besonders geeignete Ester sind die der Essig- oder Propionsäure.

Die Konzentrationen richten sich nach der Löslichkeit von $SO_2$ im Lösungsmittel; im allgemeinen liegen sie bei 0,1 bis 50, bevorzugt bei 1 bis 10 Gew.-%. Günstig ist es aber, Schwefeldioxid als Lösung in einem der oben beschriebenen Carbonsäureester einzusetzen. Schwefeldioxid wird - wie gesagt- in sehr geringen Mengen verwendet, vor allem verglichen mit durch Protonensäuren sauer katalysierten Hydroxylierungen.

Die Umsetzung findet im allgemeinen bei 20 bis 200°C statt; bevorzugt bei Temperaturen von 40 bis 180°C.

Das erfindungsgemäße Verfahren läßt sich für die Kernhydroxylierung von Phenol wie von substituierten Phenolen sowie deren Monoäthern durchführen. So lassen sich z.B. Alkylabkömmlinge von Phenol hydroxylieren, wie Kresole, Äthyl- oder Butylphenole, als auch Alkoxyverbindungen, wie Anisol, ferner deren Alkyl- oder Halogenabkömmlinge, ebenso Arylphenole, wie 4-Hydroxybiphenyl.

Selbstverständlich sind auch die Halogenverbindungen des Phenols selbst oder auch Alkoxyverbindungen des Phenols selbst einsetzbar.

Der Druck ist für die Umsetzung nicht wesentlich; im allgemeinen wird Normaldruck verwendet. Ein leichter Überdruck bis etwa 2 bar wirkt sich nicht ungünstig aus.

Ist die Para-Position zur OH-Gruppe durch einen Substituenten, wie beispielsweise eine Methylgruppe. besetzt, kann die neue Hydroxylgruppe einmal orthoständig zur OH-Gruppe, zum anderen aber orthoständig zur $CH_3$-Gruppe eintreten. Die resultierenden Produkte sind dann in 4-Stellung substituierte Brenzkatechine bzw. Resorcine.

Die erfindungsgemäss zu verwendenden Wasserstoffperoxidlösungen in höher siedenden Lösungsmitteln, deren Wassergehalt höchstens 1 Gew.-%, bevorzugt <0,5 Gew.-%, beträgt werden nach dem Verfahren der DE-Patentanmeldung P 33 08 740.7 hergestellt. Es handelt sich um Lösungsmittel, die keine oder nur solche Azeotrope mit Wasser bilden, die oberhalb des Siedepunktes von Wasser stoffperoxid sieden, bezogen auf Normaldruck.

Zu diesen Lösungsmitteln gehören Phosphorverbindungen der Formel

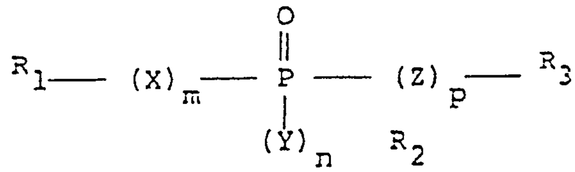

wobei X, Y und Z für ein O-Atom oder eine N-($C_1$-$C_8$)-Alkylgruppe bzw. für eine N-($C_4$-$C_7$)-Cycloalkylgruppe steht, ferner n, m und p die Zahl 0 oder 1 bedeuten, sowie $R_1$, $R_2$ und $R_3$ gradkettige oder verzweigte $C_1$-$C_8$-Alkyl- bzw. $C_4$-$C_6$-Cycloalkylreste darstellen, die gegebenenfalls durch Halogen, Hydroxyl-, $C_1$-$C_4$-Alkoxy, CN-, oder Phenylgruppen substituiert sein können.

Vor allem eignen sich Trialkylphosphate mit $C_1$-$C_8$-Alkylgrup-pen, bevorzugt Triäthylphosphat, zur Herstellung der erfindungsgemäss einzusetzenden organischen Lösungen von Wasserstoffperoxid.

Auch Ester aromatischer Carbonsäuren mit der Strukturformel

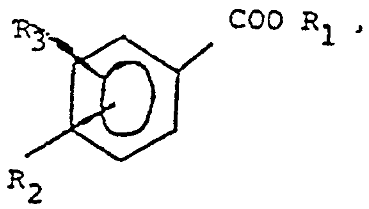

worin $R_1$ $CH_3$, $C_2H_5$, n-$C_3H_7$, i-$C_3H_7$, n-$C_4H_9$, i-$C_4H_9$, tert. $C_4H_9$, sek. $C_4H_9$, $R_2$ und $R_3$ die gegenüber Wasserstoffperoxid inerten Substituenten H, Cl, F, Alkyl wie für $R_1$ definiert, $CH_3O$, $C_2H_5O$, $COOR_4$ ($R_4$=$R_1$) bedeuten und $R_2$ und $R_3$ in beliebigen Positionen zur $COOR_1$-Gruppierung stehen kann, eignen sich ausgezeichnet für die Erfindung. So erwiesen sich besonders Phthalsäureester, bevorzugt Phthalsäurediäthylester, als sehr günstig für die einzusetzenden Wasserstoffperoxidlösungen.

Ferner können Lactame der allgemeinen Formel

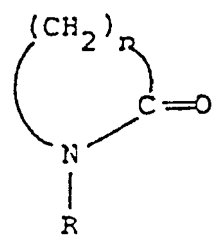

in der R einen gradkettigen oder verzweigten $C_1$ - $C_4$ Alkylrest bedeutet, der gegebenenfalls durch Halogen, Hydroxyl - oder $C_1$ - $C_3$ - Alkylreste substituiert sein kann, und n die Zahl 2 bis 5 bedeutet, eingesetzt werden.

Sehr gute Ergebnisse wurden hier mit N-Alkylpyrolidonen mit $C_1$-$C_4$-Alkylgruppen, besonders mit N-Methylpyrolidon, erzielt.

Es stellte sich auch heraus, dass tetrasubstituierte Harnstoffe als Lösungsmittel verwendet werden können in der Formel

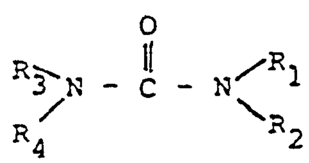

in der $R_1$, $R_2$, $R_3$ und $R_4$ $C_1$ - $C_6$ - Alkylgruppen bedeuten, wobei Harnstoffe, bei denen $R_1$, $R_2$, $R_3$ und $R_4$ einander gleich sind, bevorzugt verwendet werden.

Sehr gut erwiesen sich als höhersiedende Lösungsmittel Tetramethyl-, Tetraäthyl- und Tetrabutylharnstoff.

Wasserstoffperoxid wird als wäßrige Lösung mit 3 bis 90 Gew.-% Wasserstoffperoxid, bevorzugt 30 - 85 Gew.-% bei der Herstellung seiner organischen Lösungen eingesetzt.

Als Stabilisatoren können für Wasserstoffperoxid übliche Stabilisatoren, z.B. die in ULLMANN, Enzyklopädie der technischen Chemie, Bd. 17, 4. Aufl., Seite 709, genannten, eingesetzt werden.

Wird Schwefeldioxid als Katalysator verwendet, so ist das Molverhältnis von Phenol oder den obengenannten Phenolderivaten zu 1 Mol Wasserstoffperoxid bevorzugt 5 bis 15, vor allem aber 10 Mol Phenol oder Phenolderivat zu 1 Mol Wasserstoffperoxid.

Auf Grund der sehr geringen Katalysatormenge ist die Abtrennung des Katalysators meistens unnötig. Dies ist ein grosser Vorteil des beschriebenen Verfahrens.

Ferner ist bei Verwendung von Schwefeldioxid als Katalysator infolge der kurzen Reaktionsdauer die Raum-Zeit-Ausbeute sehr günstig. Es genügen dadurch kleine Reaktionsvolumina. Auch die Gefahr möglicher Zersetzungen wird vermieden.

Die Erfindung wird in den folgenden Beispielen näher erläutert:

**Beispiel 1**

54,7 g (0,5 Mol) p-Kresol werden auf 94°C erwärmt. Zu der gerührten Schmelze werden 0,73 g einer 1.3 gew.-%igen Lösung von $SO_2$ in Essigsäure-n-propylester gegeben und anschliessend 7,17 g einer 23,7 gew.-%igen wasserfreien Lösung von $H_2O_2$ in Triäthylphosphat (0,05 Mol).

Die Temperatur in der Reaktionslösung steigt danach auf 134°C. Nach Abklingen der Exotherme wird nach 10 min ein $H_2O_2$-Umsatz von 95,3 % bestimmt. Im Reaktionsgemisch sind dann 3,52 g (56,7 mMol) 4-Methylbrenzkatechin und 0,64 g (10,3 mMol) 4-Methylresorcin enthalten, was einer Ausbeute von 70.3 %, bezogen auf umgesetztes $H_2O_2$ entspricht.

**Beispiel 2**

94,1 g (1,0 Mol) Phenol werden auf 100°C erwärmt. Zu der gerührten Schmelze setzt man 0,4 g einer 4,85 gew.%igen Lösung von $SO_2$ in Essigsäure-iso-propylester zu und anschliessend 13,9 g einer 24,45 gew.-%igen wasserfreien Lösung von $H_2O_2$ in Triäthylphosphat ( = 0,1 Mol). Die Temperatur der Reaktionsmischung erhöht sich daraufhin auf 135°C.

Nach Abklingen der Exotherme wird nach 20 min ein $H_2O_2$-Umsatz von 91,4 ermittelt. Das Reaktionsgemisch enthält dann 5,20 g (47,2 mMol) Brenzkatechin und 2,54 g (23,1 mMol) Hydrochinon, was einer Gesamtausbeute an Dihydroxybenzolen von 76,9 %, bezogen auf umgesetztes $H_2O_2$, entspricht.

**Beispiel 3**

94,1 g (1,0 Mol) Phenol werden auf 100°C erhitzt. Zu der gerührten Schmelze setzt man 0,4 g einer 4,85 gew.%igen Lösung von Schwefeldioxid in Essigsäure-iso-propylester zu und anschliessend 16,04 g einer 21,19 gew.-%igen wasserfreien Lösung von $H_2O_2$ in Phthalsäure-diäthylester ( =0,1 Mol). Die Temperatur der Reaktionsmischung erhöht sich auf 147°C.

Nach Abklingen der Exotherme wird nach 5 min ein $H_2O_2$-Umsatz von 96,03 % bestimmt. Im Reaktionsgemisch sind dann 5,54 g (50,3 mMol) Brenzkatechin und 2,55 g (23,2 mMol) Hydrochinon enthalten, was einer Gesamtausbeute an Dihydroxybenzolen von 76,5 %, bezogen auf umgesetzte $H_2O_2$, entspricht.

**Beispiel 4**

75,1 g (0,5 Mol) 4-tert.-Butylphenol werden auf 102°C erwärmt und zu der gerührten Schmelze 0,73 g einer 1,3 gew.-%igen Lösung von $SO_2$ in Essigsäure-iso-propylester zugegeben. Anschliessend werden 8,02 g einer 21,19 gew.-%igen wasserfreien Lösung von $H_2O_2$ in Phthalsäurediäthylester zugegeben (0,05 Mol). Die Temperatur in der Reaktionslösung erhöht sich auf 132°C. Nach Abklingen der Exotherme wird nach 20 min ein Wasserstoffperoxidumsatz von 97,5 bestimmt.

Im Reaktionsgemisch sind dann 7,01 g (42,2 mMol) t-Butylbrenzkatechin enthalten, was einer Ausbeute von 86,6 %, bezogen auf umgesetztes $H_2O_2$, entspricht.

**Patentansprüche**

1. Verfahren zur Herstellung von Kernhydroxylierungsprodukten der entsprechenden Phenole oder Phenoläther durch Umsetzung derselben mit Lösungen von Wasserstoffperoxid, die einen Wassergehalt von höchstens 1 Gew.-%, bevorzugt weniger als 0,5 Gew.%, haben und mit organischen Lösungsmitteln hergestellt worden sind, die mit Wasser keine oder nur solche Azeotrope bilden, die oberhalb des Siedepunktes von Wasserstoffperoxid sieden, bezogen auf Normaldruck, in Gegenwart eines Katalysators bei einem Molverhältnis von phenol bzw. Phenoläther zu Wasserstoffperoxid im Bereich von 5 bis 20, dadurch gekennzeichnet, daß man die Umsetzung mit Schwefeldioxid als Katalysator vornimmt, wobei man 0,0005 bis 0,01 Mol Schwefeldioxid auf 1 Mol Wasserstoffperoxid verwendet.

2. Verfahren nach Anspruch 1, dadurch gekennzeichnet, daß man als Katalysator Schwefeldioxid in Lösung von Alkyl- oder Cycloalkylestern der Essig- oder Propionsäure mit einer Gesamtkohlenstoffzahl von 4 - 8 einsetzt.

3. Verfahren nach Anspruch 1 und 2, dadurch gekennzeichnet, daß man Schwefeldioxid in Lösung von Essigsäure-n- oder iso-propylester einsetzt.

4. Verfahren nach Anspruch 1 bis 3, dadurch gekennzeichnet, daß als Wasserstoffperoxidlösungen solche in Phosphorverbindungen der Formel

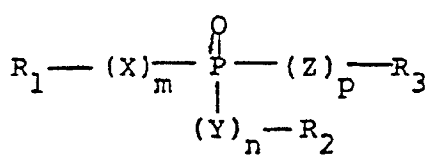

wobei X, Y und Z für ein O-Atom oder eine N-($C_1$-$C_8$)-Alkylgruppe bzw. für eine N-($C_4$-$C_7$)-Cycloalkylgruppe steht, ferner n, m und p die Zahl 0 oder 1 bedeuten, sowie $R_1$, $R_2$ und $R_3$ gradkettige oder verzweigte $C_1$-$C_8$-Alkyl- bzw. $C_4$-$C_6$-Cycloalkylreste darstellen, die gegebenenfalls durch Halogen, Hydroxyl, $C_1$-$C_4$-Alkoxy, CN, oder Phenyl substituiert sein können, eingesetzt werden.

5. Verfahren nach Anspruch 1 bis 4, dadurch gekennzeichnet, daß Wasserstoffperoxidlösungen in Trialkylphosphaten mit $C_1$ bis $C_8$-Alkylgruppen, bevorzugt Triäthylphosphat und Trioctylphosphat, eingesetzt werden.

6. Verfahren nach Anspruch 1 bis 3, dadurch gekennzeichnet, daß als Wasserstoffperoxidlösungen solche in aromatischen Carbonsäureestern der Formel

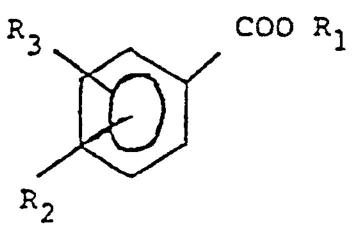

$R_1$ $CH_3$, $C_2H_5$, n-$C_3H_7$, i-$C_3H_7$, n-$C_4H_9$, i-$C_4H_9$, tert. $C_4H_9$ oder, sek. $C_4H_9$, $R_2$ und $R_3$ H, Cl, F, Alkyl wie für $R_1$ definiert, $CH_3O$, $C_2H_5O$ oder $COOR_4$ ($R_4 = R_1$) bedeuten und $R_2$ und $R_3$ in beliebigen Positionen zur $COOR_1$-Gruppierung stehen können, eingesetzt werden.

7. Verfahren nach Anspruch 1 bis 3 und 6, dadurch gekennzeichnet, daß Wasserstoffperoxidlösungen in Phthalsäuredialkylestern, bevorzugt Phthalsäurediäthylester, eingesetzt werden.

8. Verfahren nach Anspruch 1 bis 3, dadurch gekennzeichnet, daß als Wasserstoffperoxidlösungen solche in Lactamen der Formel

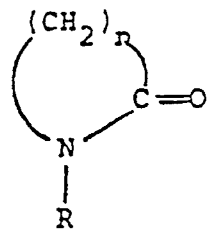

in der R einen gradkettigen oder verzweigten $C_1$-$C_4$-Alkylrest bedeutet, der gegebenenfalls durch Halogen, Hydroxyl- oder $C_1$-$C_3$-Alkylreste substituiert sein kann, und n die Zahl 2 bis 5 bedeutet, eingesetzt werden.

9. Verfahren nach Anspruch 1 bis 3 und 8, dadurch gekennzeichnet, daß Wasserstoffperoxidlösungen in N-Alkylpyrolidonen mit $C_1$-$C_4$-Alkylgruppen, bevorzugt in N-Methylpyrolidon, eingesetzt werden.

10. Verfahren nach Anspruch 1 bis 3, dadurch gekennzeichnet, daß als Wasserstoffperoxidlösungen solche in tetrasubstituierten Harnstoffen der Formel

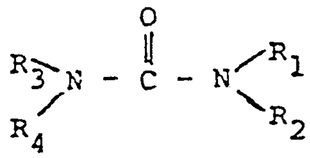

in der $R_1$, $R_2$, $R_3$ und $R_4$ $C_1$-$C_6$-Alkylgruppen bedeuten, eingesetzt werden wobei Harnstoffe, bei denen $R_1$, $R_2$, $R_3$ und $R_4$ einander gleich sind, bevorzugt sind.

11. Verfahren nach Anspruch 1 bis 3 und 10, dadurch gekennzeichnet, daß Wasserstoffperoxidlösungen in Tetramethyl-, Tetraäthyl- oder Tetrabutylharnstoff eingesetzt werden.

## Claims

1. A process for the production of nuclear hydroxylation products of the corresponding phenols or phenol ethers by reaction of said phenols or phenol ethers with solutions of hydrogen peroxide having a water content of at most 1 % by weight, preferably less than 0,5 % by weight and produced using organic solvents which, with water, form no azeotropes or only those azeotropes which boil above the boiling point of hydrogen peroxide at normal pressure in the presence of a catalyst at a molar ratio of phenol or phenol ether to hydrogen peroxide in the range of from 5 to 20, characterized in that the reaction is carried out with sulfur dioxide as catalyst wherein 0,0005 to 0,01 mol of sulfur dioxide based on 1 mol of hydrogen peroxide are used.

2 . A process according to claim 1, characterised in that sulphur dioxide in a solution of alkyl or cycloalkyl esters of acetic or propionic acid having a total carbon number of from 4 to 8 is used as catalyst.

3 . A process according to claims 1 and 2, characterised in that sulphur dioxide in a solution of acetic acid nor iso-propyl ester is used.

4 . A process according to claims 1 to 3, characterised in that hydrogen peroxide solutions in phosphorus compounds corresponding to the formula

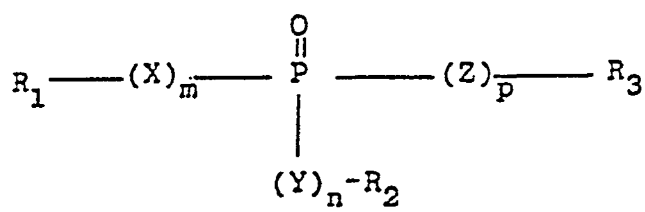

wherein X, Y and Z represent an O-atom or a N-($C_1$-$C_8$)-alkyl group or a N-($C_4$-$C_7$)-cycloalkyl group, also n, m and p represent the number 0 or 1 and $R_1$, $R_2$ and $R_3$ represent straight-chained or branched $C_1$-$C_8$-alkyl or $C_4$-$C_6$-cycloalkyl radicals which may optionally be substituted by halogen, hydroxyl, $C_1$-$C_4$-alkoxy, CN or phenyl are used as hydrogen peroxide solutions.

5. A process according to claims 1 to 4, characterised in that hydrogen peroxide solutions in trialkyl phos-

phates containing $C_1$-$C_8$ alkyl groups preferably triethyl phosphate and trioctyl phosphate are used.

6. A process according to claims 1 to 3, characterised in that hydrogen peroxide solutions in aromatic carboxylic acid esters corresponding to the formula

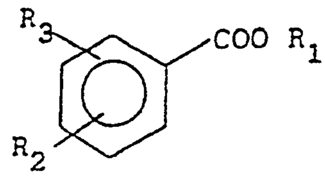

wherein $R_1$ represents $CH_3$, $C_2H_5$, n-$C_3H_7$, i-$C_3H_7$, n-$C_4H_9$, i-$C_4H_9$, tert. $C_4H_9$ or sec. $C_4H_9$, $R_2$ and $R_3$ represent H, Cl, F, alkyl as defined for $R_1$, $CH_3O$, $C_2H_5O$ or $COOR_4$ ($R_4 = R_1$) and $R_2$ and $R_3$ can stand in any positions relative to the $COOR_1$ grouping, are used as hydrogen peroxide solutions.

7. A process according to claims 1 to 3 and 6, characterised in that hydrogen peroxide solutions in phthalic acid dialkyl esters, preferably phthalic acid diethyl ester are used.

8. A process according to claims 1 to 3, characterised in that hydrogen peroxide solutions in lactams corresponding to the formula

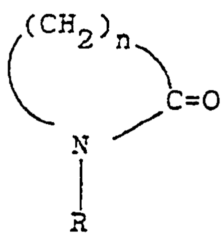

in which R represents a straight-chained or branched $C_1$-$C_4$-alkyl radical which may optionally be substituted by halogen, hydroxyl or $C_1$-$C_3$- alkyl radicals and n represents the number from 2 to 5 are used as hydrogen peroxide solutions.

9. A process according to claims 1 to 3 and 8, characterised in that hydrogen peroxide solutions in N-alkyl pyrolidones with $C_1$-$C_4$- alkyl groups, preferably in N-methyl pyrolidone are used.

10. A process according to claims 1 to 3, characterised in that hydrogen peroxide solutions in tetrasubstituted ureas corresponding to the formula

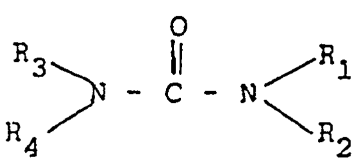

in which $R_1$, $R_2$, $R_3$ and $R_4$ represent $C_1$-$C_6$- alkyl groups are used as hydrogen peroxide solutions, ureas in which $R_1$, $R_2$, $R_3$, and $R_4$ are identical to one another being preferred.

11. A process according to claims 1 to 3 and 10, characterised in that hydrogen peroxide solutions in tetramethyl tetraethyl or tetrabutyl urea are used.

## Revendications

1. Procédé pour la fabrication de produits de l'hydroxylation nucléaire des phénols ou éthers phénoliques correspondants, par réaction de ceux-ci avec des solutions de peroxyde d'hydrogène qui ont une teneur en eau de 1 % en poids au maximum et mieux inférieure à 0,5 % en poids et qui ont été préparées avec des solvants organiques quine forment avec l'eau aucun azéotrope, ou seulement un azéotrope dont le point d'ébullition est supérieur à celui du peroxyde d'hydrogène, calculé à la pression normale, en présence d'un catalyseur et en utilisant un rapport molaire entre le phénol ou l'éther phénolique et le peroxyde d'hydrogène de 5 à 20, caractérisé en ce que l'on assure la réaction avec l'anhydride sulfureux comme catalysateur et que l'on utilise de

0,0005 à 0,01 mol de l'anhydride sulfureux calculé sur une mole de peroxyde d'hydrogène.

2. Procédé suivant la revendication 1, caractérisé en ce que l'on utilise, comme catalyseur, l'anhydride sulfureux en solution dans des esters alcoylés ou cycloalcoylés des acides acétique ou propionique avec un nombre total de carbones de 4 à 8.

3. Procédé suivant les revendications 1 à 2, caractérisé en ce qu'on utilise l'anhydride sulfureux en solution dans le n- ou l'iso-propylester de l'acide acétique.

4. Procédé suivant les revendications 1 à 3, caractérisé en ce que l'on utilise comme solutions de peroxyde d'hydrogène, des solutons dans des composés du phosphore répondant à la formule:

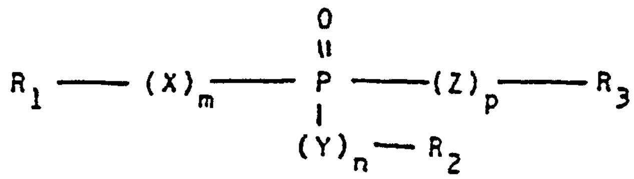

ou X, Y et Z représentent un atome O, ou un groupe alcoyl N-($C_1$-$C_8$) ou un groupe cycloalcoyl N-($C_4$-$C_7$), et n, m, et p, le chiffre 0 ou 1, $R_1$, $R_2$ et $R_3$ représentant des restes alcoyl en $C_1$ à $C_8$, ou cycloalcoyl en $C_4$ à $C_6$, qui peuvent être éventuellement substitués par des halogènes, groupes hydroxyl-, alcoxy en $C_1$ à $C_4$, CN, ou phényl.

5. Procédé suivant les revendications 1 à 4, caractérisé en ce qu'on utilise des solutions de peroxyde d'hydrogène dans des trialkylphosphate avec des groupes alcoyl en $C_1$ à $C_8$, de préférence les triéthyl- et trioctyl-phosphate.

6. Procédé suivant les revendications 1 à 3 caractérisé en ce que l'on utilise, comme solutions de peroxyde d'hydrogène, celles qui sont préparées dans des esters d'acides carboxyliques aromatiques répondant à la formule:

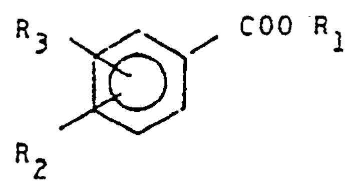

où R représente $CH_3$, $C_2H_5$, n-$C_3H_7$, i-$C_3H_7$, n-$C_4H_9$, i-$C_4H_9$, $C_4H_9$ tert., $C_4H_9$ sec., $R_2$ et $R_3$ H, Cl, F, alcoyl tels que ceux définis pour $R_1$, $CH_3O$, $C_2H_5O$, $COOR_4$ ($R_4 = R_1$), $R_2$ et $R_3$ pouvant se trouver dans des positions quelconques par rapport aux groupements $COOR_1$.

7. Procédé suivant les revendications 1 à 3 et 6 , caractérisé en ce qu'on utilise des solutions de peroxyde d'hydrogène dans des esters dialcoylés de l'acide phtalique, de préférence de diéthylester de l'acide phtalique.

8. Procédé suivant l'une des revendications 1 à 3, caractérisé en ce qu'on utilise, comme solutions de peroxyde d'hydrogène, celles qui sont préparées dans des lactames répondant à la formule:

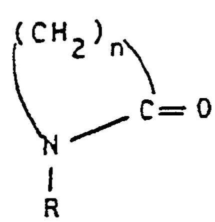

où R représente un reste alcoyle en $C_1$ à $C_4$, à chaine droite ou ramifiée, qui peut être éventuellement substitué par un halogène, un hydroxyl, ou des restes alcoyl en $C_1$ à $C_3$, et n représente l'un des chiffres 2 à 5.

9. Procédé suivant les revendications 1 à 3 et 8, caractérisé en ce que l'on utilise des solutions de peroxyde d'hydrogène dans des N-alcoylpyrrolidones avec des groupes alcoyl en $C_1$ à $C_4$ et de préférence dans la N-méthylpyrrolidone.

10. Procédé suivant les revendications 1 à 3, caractérisé en ce que l'on utilise comme solutions de peroxyde d'hydrogène celles qui sont préparées dans des urées tétra-substituées répondant à la formule:

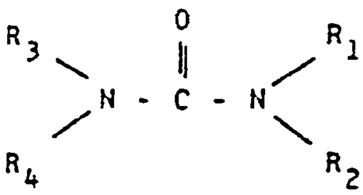

où $R_1$, $R_2$, $R_3$ et $R_4$ représentent des groupes alcoyl en $C_1$ à $C_6$ les urées dans lesquelles $R_1$, $R_2$, $R_3$ et $R_4$ sont semblables étant recommandées.

11. Procédé suivant les revendications 1 à 3 et 10, caractérisé en ce que l'on utilise des solutions de peroxyde d'hydrogène dans la tétraméthyl-, tétraéthyl- ou tétrabutylurée.